# EUROPEAN PATENT APPLICATION

(11) **EP 3 943 051 A1**
(43) Date of publication of application: **26.01.2022**
(21) Application number: 21186062.2
(22) Date of filing: 16.07.2021
(51) Int. Cl.: A61F 2/46

(54) **HANDLE ASSEMBLY FOR A MEDICAL DEVICE INSTRUMENT**

(30) Priority: 21.07.2020 US 202063054391 P
(71) Applicant: Shukla Medical, Saint Petersburg, Florida 33709 (US)
(72) Inventor: SWEITZER, Zachary Robert, Keyport, NJ 07735 (US)
(74) Representative: Cameron Intellectual Property Ltd

(57) **Abstract**

A handle assembly (100) for a medical device instrument including a handle (104) having a shaft (106) including a proximal end (108), and a distal end (110). The handle further includes a hand grip (112) having first (114) and second (116) ends affixed to the proximal end of the shaft and a longitudinal axis (B) extending substantially transverse to the longitudinal axis (A) of the shaft. At least one of the first and second ends of the hand grip includes a ring (118) having a longitudinal through axis (C) spaced from and substantially parallel to the longitudinal axis of the shaft. The ring is sized sufficiently to receive a user's finger to enable the handle to be rotated. The handle assembly further includes a quick connect (120) at the distal end of the shaft for releasably connecting the handle assembly to a shaft (122) of the medical device instrument.

## Description

### BACKGROUND OF THE DISCLOSURE

The exemplary embodiments of present invention relate generally to a handle assembly for a surgical tool and, more specifically, to a handle assembly for a medical device instrument for extracting an implant from bone including, without limitation, a glenosphere implant.

Certain implant extractor tools have a handle including a hand grip that may be grasped by a user, e.g., a surgeon, and rotated to a desired position which is comfortable to the user when exerting an extraction force on the handle. The extent to which the handle may be rotated is limited by the extent to which the user's wrist can rotate, which is normally about 180 degrees. However, if the user suffers from a limited range of wrist rotation, the user may be required to rotate the handle a certain degree, release the handle, re-grasp the handle and continue to rotate the handle to a comfortable extraction position, or be required to regrip multiple times if the handle needs to be rotated more than 180 degrees. Additionally, it is important to make the instruments used in an operating room as user-friendly and time-efficient as possible. It is well understood that the shorter the time a patient is under anesthesia, the lesser the likelihood the patient will suffer adverse effects due to the anesthesia.

### BRIEF SUMMARY OF THE DISCLOSURE

In accordance with an exemplary embodiment there is provided a handle assembly for a medical device instrument comprising a handle including a shaft having a proximal end and a distal end. The handle further includes a hand grip having first and second ends affixed to the proximal end of the shaft and a longitudinal axis extending substantially transverse to a longitudinal axis of the shaft. At least one of the first and second ends of the hand grip includes a ring having a longitudinal through axis spaced from and substantially parallel to the longitudinal axis of the shaft, wherein the ring is sized sufficiently to receive a finger. The handle assembly further comprises a quick connect at the distal end of the shaft for releasably connecting the handle assembly to a shaft of a medical device instrument.

According to an aspect, the central axial opening is an elongated opening. According to another aspect, the housing includes a second axial opening for receiving a first locking element, and a third axial opening for receiving a second locking element. According to another aspect, the housing further includes a laterally facing counterbore.

According to an aspect, the quick connect comprises a housing including a central axial opening for receiving a shaft of the medical device instrument, and a lateral recess having a laterally facing opening. According to another aspect, the quick connect comprises a locking mechanism for releasably engaging with the shaft of the medical device instrument. According to another aspect, the locking mechanism comprises a locking member having first and second ends and an elongated through hole for receiving a shaft of the medical device instrument, and a biasing member biasing the second end of the locking member. According to another aspect, the locking member comprises a lateral recess. According to another aspect, the locking member comprises a pair of opposing lateral recesses.

According to an aspect, the first end of the hand grip includes the ring and a lateral most end of the ring extends further laterally from the shaft than a lateral most end of the second end of the hand grip. According to another aspect, the ring has an overall diameter larger than an overall thickness of the hand grip.

In accordance with another exemplary embodiment there is provided a handle assembly for a medical device instrument comprising a handle including a shaft having a proximal end, a distal end, and a longitudinal axis. The handle further includes a hand grip affixed to the proximal end of the shaft. The hand grip has a longitudinal axis extending substantially transverse to the longitudinal axis of the shaft. The hand grip further comprises a first end that includes a ring having a through hole sized sufficiently to receive a finger and a through axis spaced from and substantially parallel to the longitudinal axis of the shaft, and a second end opposite the first end. The handle assembly further comprises a quick connect at the distal end of the shaft for releasably connecting the handle assembly to a shaft of the medical device instrument. The quick connect includes a housing having a central axial elongated opening for receiving the shaft of the medical device instrument, a second axial opening for receiving a first locking element, a third axial opening for receiving a second locking element, and a lateral recess having a laterally facing opening.

According to an aspect, a lateral most end of the ring extends further laterally from the shaft than a lateral most end of the second end of the hand grip. According to another aspect, the quick connect further includes a locking mechanism structured to releasably engage with the shaft of the medical device instrument and slidable within the lateral recess of the housing. According to another aspect, the locking mechanism comprises a locking member and a biasing member. According to another aspect, the locking member has first and second ends, an elongated through hole for receiving the shaft of the medical device instrument, and a pair of opposing lateral recesses for receiving respective first and second locking elements. According to another aspect, the locking mechanism further comprises a biasing member mounted within a counterbore of the housing and biasing the second end of the locking member.

In accordance with another exemplary embodiment there is provided a handle assembly for a medical device instrument comprising a handle including a shaft having a proximal end, a distal end, and a longitudinal axis. The handle further includes a hand grip affixed to the proximal end of the shaft. The hand grip has a longitudinal axis extending substantially transverse to the longitudinal axis of the shaft. The hand grip further comprises a first end and a second end opposite the first end. The first end includes a ring having a through hole and a through axis spaced from and substantially parallel to the longitudinal axis of the shaft. The ring is sized sufficiently to receive a finger and has an overall diameter larger than an overall thickness of the hand grip. The handle assembly further comprises a quick connect at the distal end of the shaft for releasably connecting the handle assembly to a shaft of the medical device instrument.

According to an aspect, the handle assembly further comprises a locking mechanism structured to releasably engage with the shaft of the medical device instrument, and slidable within a lateral recess of the housing. According to another aspect, the locking mechanism has first and second ends, an elongated through hole for receiving the shaft of the medical device instrument, and a pair of opposing lateral recesses for receiving respective first and second locking elements. According to another aspect, the locking mechanism further comprises a biasing member mounted within a counterbore of the housing and biasing the second end of the locking member.

As a result of the exemplary embodiments, there is provided a handle assembly for a medical device instrument including a hand grip that may be easily placed into a desired position by insertion of the user's finger through the through hole in the ring and rotating the ring through any angle from 0 to 360 degrees.

Other features and advantages of the subject disclosure will be apparent from the following more detailed description of the exemplary embodiments.

### BRIEF DESCRIPTION OF THE SEVERAL VIEWS OF THE DRAWINGS

The foregoing summary, as well as the following detailed description of the exemplary embodiments of the subject disclosure, will be better understood when read in conjunction with the appended drawings. For the purpose of illustrating the present disclosure, there are shown in the drawings exemplary embodiments. It should be understood, however, that the subject application is not limited to the precise arrangements and instrumentalities shown.
FIG. 1 is perspective view of a handle assembly in accordance with an exemplary embodiment of the subject disclosure attached to a medical device instrument;
FIG. 2 is an exploded perspective view of the handle assembly of FIG. 1 and a medical device instrument shaft to which the handle assembly is releasably attachable;
FIG. 3 is a side view of the handle assembly and medical device instrument shaft of FIG. 2;
FIG. 4 is a perspective view of the handle assembly of FIG. 1 with certain elements shown in phantom line for purposes of clarity;
FIG. 5 is a perspective view of a handle assembly similar to FIG. 4, with certain elements omitted for purposes of clarity;
FIG. 6 is a side view of the handle assembly of FIG. 1 with certain elements omitted for purposes of clarity;
FIG. 7 is a cross-sectional perspective view of the handle assembly of FIG. 1, with certain elements omitted for purposes of clarity;
FIG. 8 is a cross-sectional side view of the handle assembly of FIG. 1 and a medical device instrument shaft engaged therewith;
FIG. 9 is a top view of the handle assembly of FIG. 1;
FIG. 10 is a front perspective view of a locking member of the handle assembly of FIG. 1; and
FIG. 11 is an enlarged perspective view of a locking element of the handle assembly of FIG. 1.

### DETAILED DESCRIPTION OF THE DISCLOSURE

Reference will now be made in detail to the various exemplary embodiments of the subject disclosure illustrated in the accompanying drawings. Wherever possible, the same or like reference numbers will be used throughout the drawings to refer to the same or like features. It should be noted that the drawings are in simplified form and are not drawn to precise scale. Certain terminology is used in the following description for convenience only and is not limiting. Directional terms such as top, bottom, left, right, above, below and diagonal, are used with respect to the accompanying drawings. The term "distal" shall mean away from the center of a body. The term "proximal" shall mean closer towards the center of a body and/or away from the "distal" end. The words "inwardly" and "outwardly" refer to directions toward and away from, respectively, the geometric center of the identified element and designated parts thereof. Such directional terms used in conjunction with the following description of the drawings should not be construed to limit the scope of the subject application in any manner not explicitly set forth. Additionally, the term "a," as used in the specification, means "at least one." The terminology includes the words above specifically mentioned, derivatives thereof, and words of similar import.

"About" as used herein when referring to a measurable value such as an amount, a temporal duration, and the like, is meant to encompass variations of ±20%, ±10%, ±5%, ±1%, or ±0.1% from the specified value, as such variations are appropriate.

"Substantially" as used herein shall mean considerable in extent, largely but not wholly that which is specified, or an appropriate variation therefrom as is acceptable within the field of art. "Exemplary" as used herein shall mean serving as an example.

Throughout the subject application, various aspects thereof can be presented in a range format. It should be understood that the description in range format is merely for convenience and brevity and should not be construed as an inflexible limitation on the scope of the subject disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

Furthermore, the described features, advantages and characteristics of the exemplary embodiments of the subject disclosure may be combined in any suitable manner in one or more embodiments. One skilled in the relevant art will recognize, in light of the description herein, that the subject disclosure can be practiced without one or more of the specific features or advantages of a particular exemplary embodiment. In other instances, additional features and advantages may be recognized in certain embodiments that may not be present in all exemplary embodiments of the present disclosure.

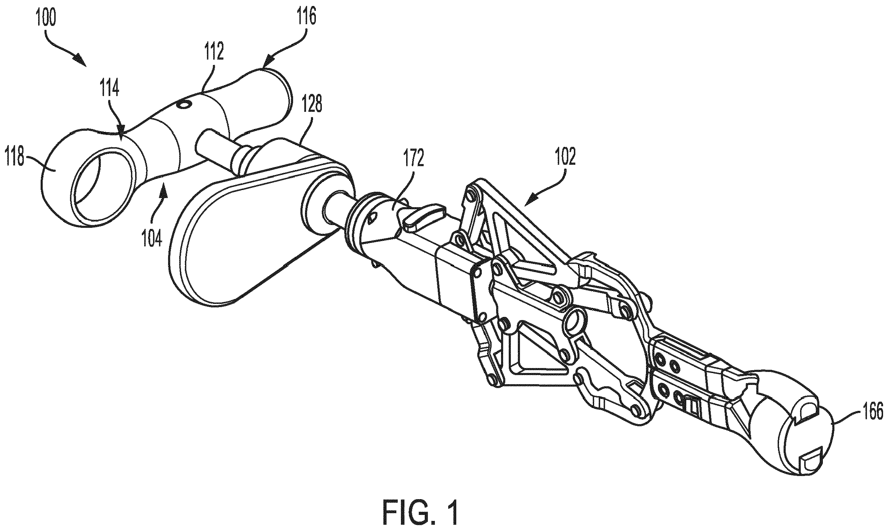

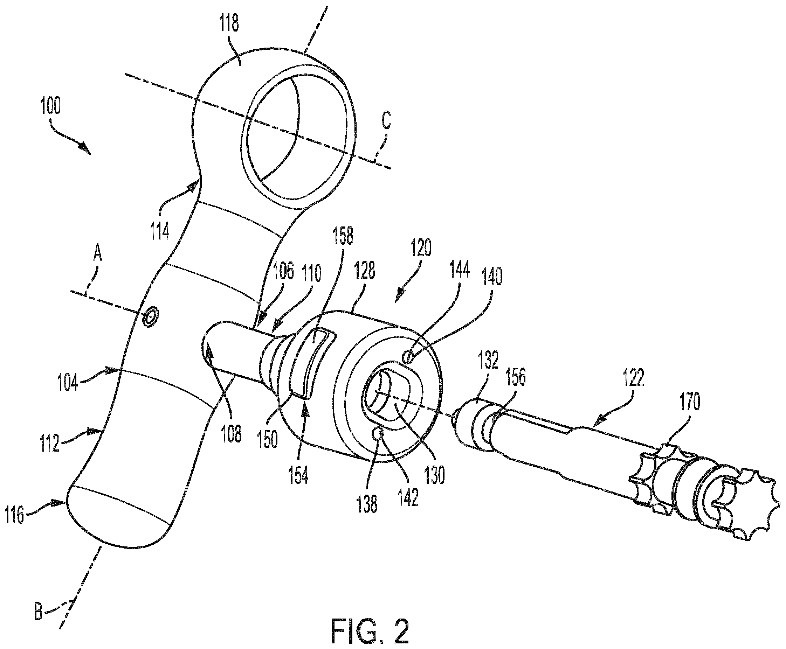

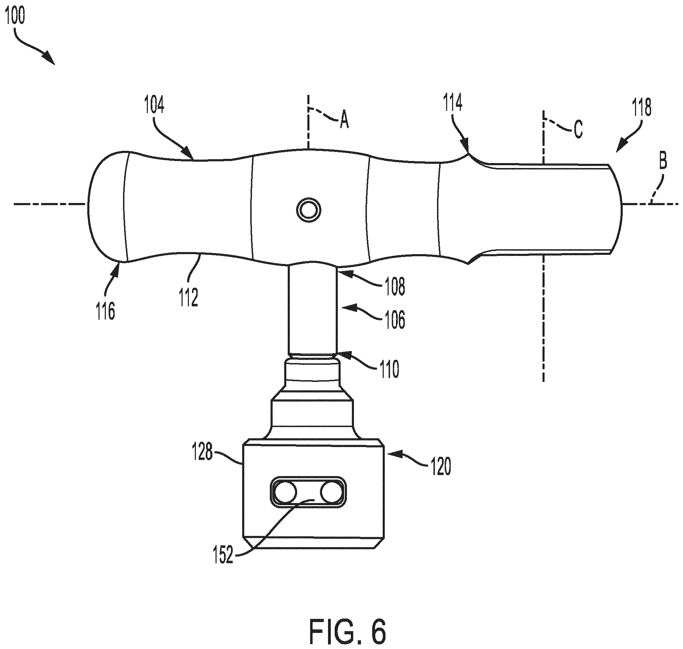

Referring now to the drawings, FIG. 1 illustrates a handle assembly 100 for a medical device instrument 102 in accordance with an exemplary embodiment of the present disclosure. FIGS. 2, 3, 6 and 9 illustrate that the handle assembly 100 comprises a handle 104 and a quick connect 120.

The handle 104 includes a shaft 106 and a hand grip 112. The shaft has a proximal end 108, a distal end 110, and a central longitudinal axis "A". The hand grip 112 has first and second ends 114, 116 and is affixed to the proximal end 108 of the shaft 106. A central longitudinal axis "B" of the hand grip extends substantially transverse to the longitudinal axis A of the shaft.

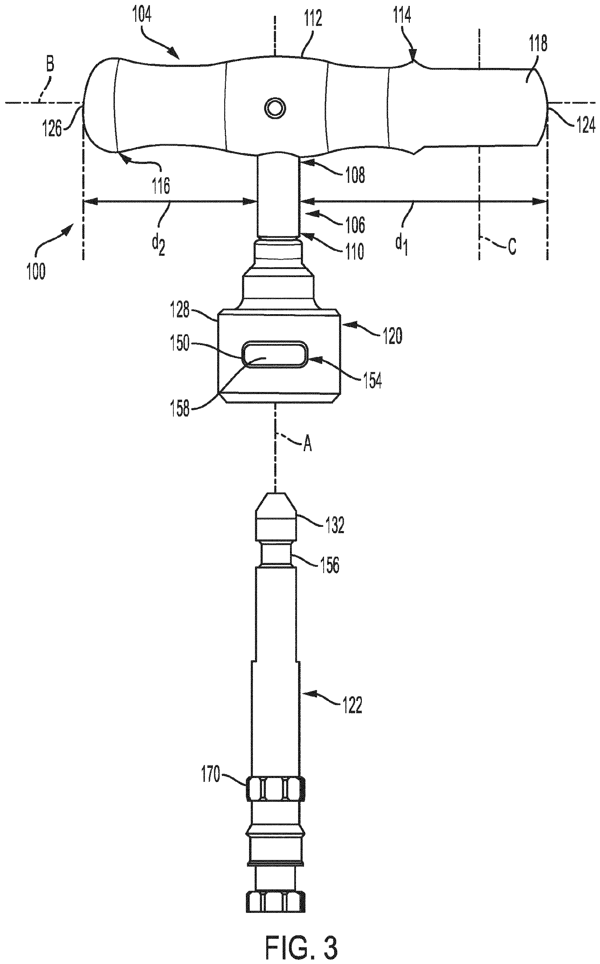

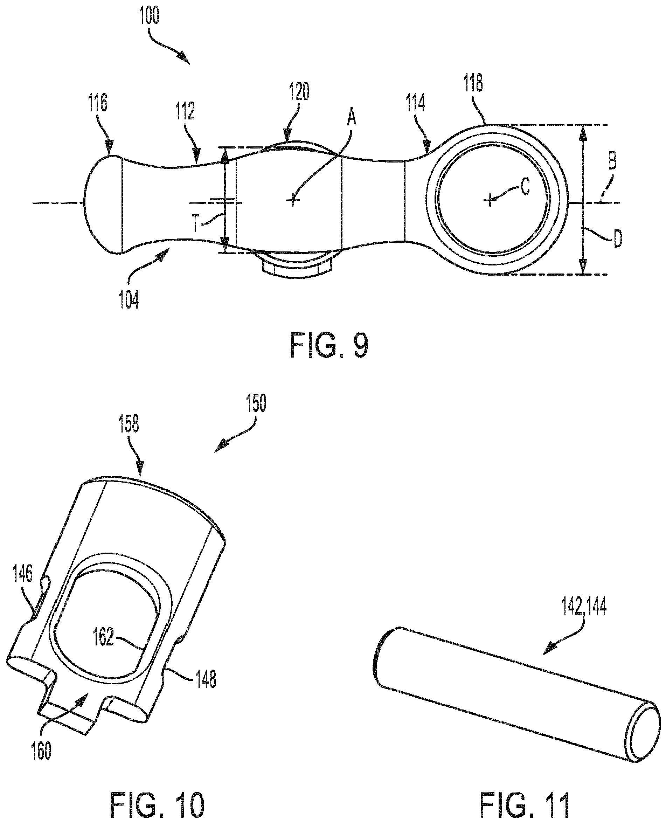

At least one of the first and second ends of the hand grip includes a ring 118. The ring has a central through axis "C" spaced from and substantially parallel to the longitudinal axis A of the shaft 106. According to an aspect, the ring 118 is sized sufficiently to receive a finger. According to an aspect, FIGS. 1-7 and 9 illustrate that the first end 114 of the hand grip 112 includes the ring 118. FIG. 3 most clearly illustrates that a lateral most end 124 of the ring extends a distance "di" further laterally from the shaft 106 or the central longitudinal axis A of the shaft than a distance "d₂" that a lateral most end 126 of the second end 116 of the hand grip extends from the shaft 106 or the central longitudinal axis A of the shaft. FIG. 9 illustrates that the ring 118 has an overall diameter "D" larger than an overall thickness or diameter "T" of the hand grip 112. It is understood that the ring 118 can alternatively be provided on the second end 116 of the hand grip 112. In addition, it is contemplated that a ring can provided at both ends of the hand grip.

The quick connect 120 is disposed at the distal end 110 of the shaft 106. The quick connect is operable for releasably connecting the handle assembly to a shaft 122 of a medical device instrument such as medical device instrument 102. FIGS. 2-11 illustrate various features of the quick connect 120. As most clearly shown in FIGS. 2, 5, 7 and 8, the quick connect 120 comprises a housing 128 including a central axial opening 130 and a lateral recess 134.

The central axial opening 130 is structured for receiving the shaft 122 of the medical device instrument 102 (FIG. 1), in particular a proximal end 132 of the shaft. According to an aspect, the central axial opening is an elongated opening, having e.g., a pair of opposing flat or linear sides.

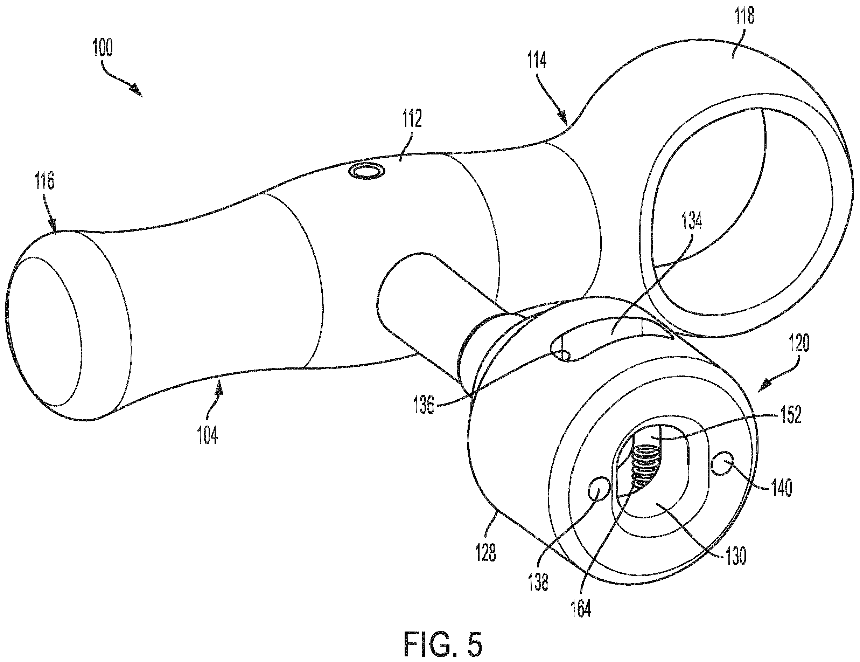

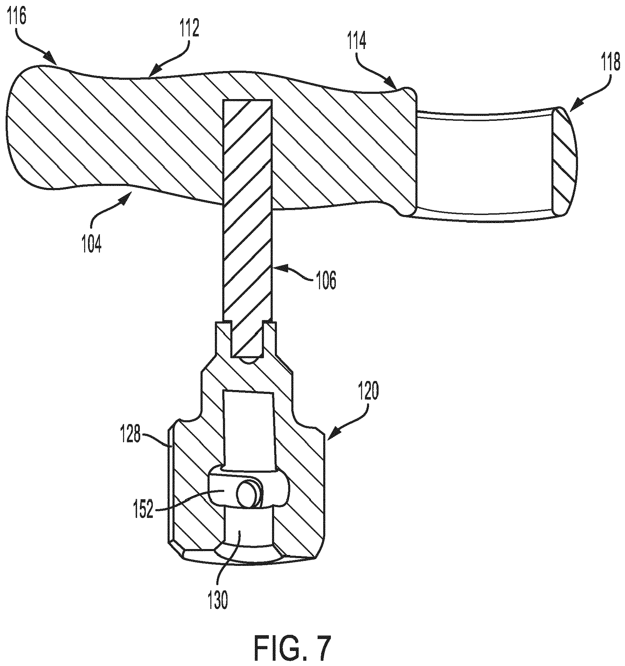

The lateral recess 134 extends in a widthwise direction of the housing and has a laterally facing opening 136. The lateral recess 134 also includes a medially facing counterbore 152, as best shown in FIGS. 5 and 7. The lateral recess receives the locking member 150, with a portion of the locking member the laterally facing opening as described in further detail below.

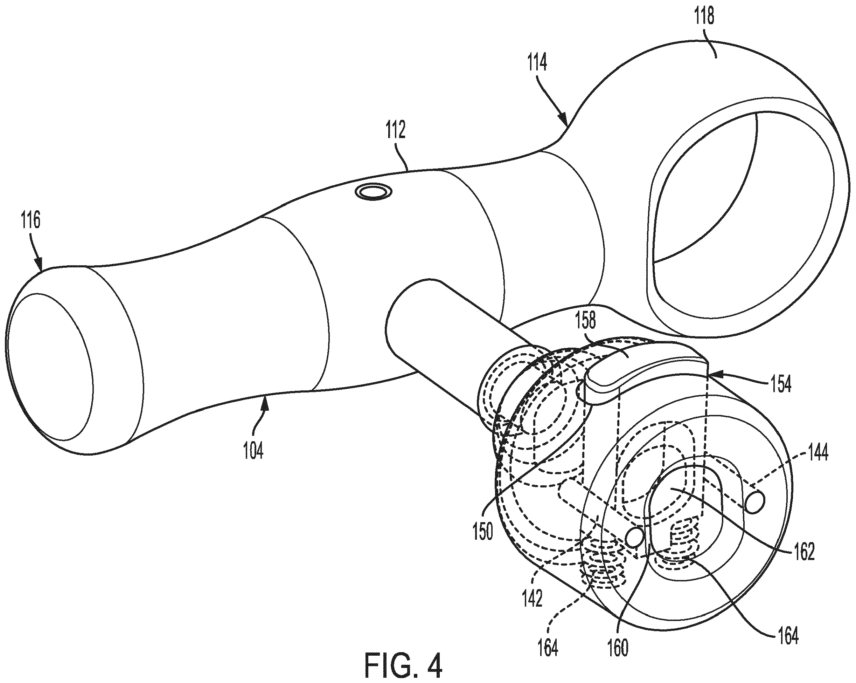

FIG. 5 additionally illustrates that the housing further includes a second axial opening 138 and a third axial opening 140. As shown in FIG. 2, the second and third axial openings 138, 140 respectively receive a first locking element 142 and a second locking element 144 (an enlarged view of which is shown in FIG. 11). The first and second locking elements 142, 144 can be constructed as elongated pins or dowels which are operable to be received in lateral recesses 146, 148 of a locking member 150 (FIGS. 4, and 10) in the manner described herebelow.

Referring to FIG. 4, the quick connect 120 comprises a locking mechanism 154. The locking mechanism includes a locking member 150 and one or more biasing members 164. FIG. 10 best illustrates the locking member 150. The locking member is structured to releasably engage with the shaft of the medical device instrument and slidable within the lateral recess 134 of the housing. According to an aspect, the locking mechanism is structured to releasably engage with a reduced diameter portion 156 of the medical device instrument shaft 122 adjacent the proximal end 132 thereof (FIGS. 2, 3 and 8). As most clearly shown in FIG. 10, the locking member has first and second ends 158, 160 and an elongated through hole 162 for receiving the shaft 122 of the medical device instrument. The elongated through hole can include opposing flats or linear sides. FIG. 10 shows that the locking member comprises at least a lateral recess or a pair of opposing lateral recesses 146, 148 for receiving respective first and second locking elements 142, 144.

As illustrated most clearly in FIG. 4, the locking mechanism may include a pair of biasing members 164 mounted within the laterally facing counterbore 152 of the lateral recess and biasing the second end 160 of the locking member 150 whereby the first end 158 of the locking member laterally protrudes through the laterally facing opening 136 of the lateral recess.

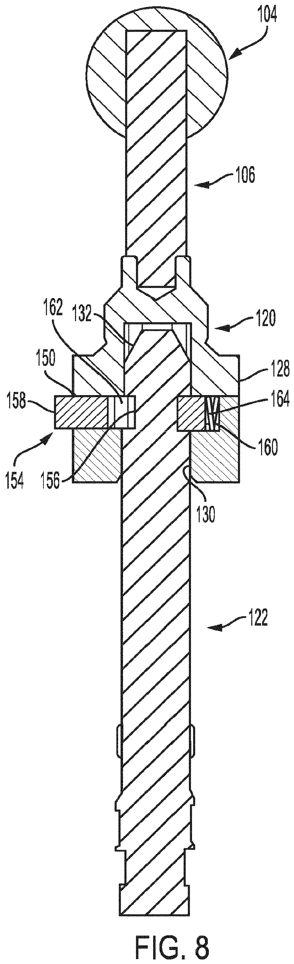

In operation, when it is desired to connect the medical device instrument 102 to the handle assembly 100 the user, e.g., a surgeon, depresses the first end 158 of the locking member 150 against the biasing force of the biasing member 164. With the first end of the locking member so depressed, the elongated through hole 162 comes into alignment with the central axial opening 130 in the housing 128, thereby permitting the proximal end 132 of the medical device instrument shaft 122 to be inserted into housing. When the proximal end of the medical device instrument shaft is fully inserted in the housing, the locking member 150 is aligned with the reduced diameter annular groove 156 of the medical device instrument. The user then releases the first end of the locking member and the biasing member biases the locking member into engagement with the reduced diameter annular groove (as shown in FIG. 8), whereby the medical device instrument shaft is axially locked in the housing.

With the medical device instrument shaft axially locked in the housing, the user may grip the hand grip 112 and rotate the handle 104 to a desired angular position in relation to the medical device instrument. Alternatively, the user may insert his or her thumb or finger through the ring 118 in the hand grip and exert a 0 to 360 or more degree turning or torque force about the longitudinal axis A of the shaft 106 to bring or rotate the handle to a desired angular position in relation to the medical device instrument or drive rotation of the medical device instrument shaft. Rotational translation between the handle assembly and the medical device instrument is prevented by engagement of an intermediate splined portion 170 (FIGS. 2 and 3) of the medical device instrument shaft 122 with a corresponding unillustrated internal splined socket provided in a housing 172 (FIG. 1) at a proximal end of the medical device instrument. With the handle positioned in the desired angular position and the splined portion 170 seated in the unillustrated internal splined socket in the housing 172, the user may grip the hand grip 112 and exert a proximally directed pulling force on the handle and thus on the medical device instrument to extract e.g., an implant 166 (FIG. 1) gripped by a distal end of the medical device instrument from bodily tissue including, without limitation, bone.

When it is desired to release the handle assembly from locking engagement with the medical device instrument, the user depresses the first end 158 of the locking member 150 until the locking member releases from engagement with the annular groove 156 of the medical device instrument shaft. With the first end of the locking member so depressed, the user removes the medical device instrument shaft from the housing.

The various exemplary embodiments of the handle assembly discussed herein provide numerous advantages over conventional handle assemblies. For example, the present handle assembly includes a ring, e.g., ring 118, to allow for substantially continuous and smooth rotation of the handle assembly, without the need for the user to release and re-grasp a conventional handle assembly to continue rotation. In other words, a ring on one end of the T-handle allows the user to insert a finger in the ring and rapidly spin the handle to control or adjust whatever other instrument to which the handle is attached. This saves time and reduces potential complications for the patient as the patient is typically under anesthesia.

Another advantage of the exemplary handle assembly is the arrangement and use of a quick connect at a distal end of the handle assembly, e.g., quick connect 120. The quick connect allows for straightforward and speedy releasable connection of the present handle assembly to a medical device instrument.

It will be appreciated by those skilled in the art that changes could be made to the exemplary embodiments described above without departing from the broad inventive concept thereof. It is to be understood, therefore, that this disclosure is not limited to the particular embodiments disclosed, but it is intended to cover modifications within the spirit and scope of the subject disclosure as defined by the appended claims.

## Claims

1. A handle assembly (100) for a medical device instrument comprising a handle (104) including a shaft (106) having a proximal end (108) and a distal end (110), and a hand grip (112) having first and second ends (114, 116) affixed to the proximal end of the shaft and a longitudinal axis (B) extending substantially transverse to a longitudinal axis (A) of the shaft, **characterized by**:
at least one of the first and second ends of the hand grip including a ring (118) having a longitudinal through axis (C) spaced from and substantially parallel to the longitudinal axis of the shaft, and wherein the ring is sized sufficiently to receive a finger; and
a quick connect (120) at the distal end of the shaft for releasably connecting the handle assembly to a shaft (122) of a medical device instrument.

2. The handle assembly of claim 1, wherein the quick connect comprises a housing (128) including:
a central axial opening (130) for receiving a shaft of the medical device instrument; and
a lateral recess (134) having a laterally facing opening (136).

3. The handle assembly of claim 2, wherein the central axial opening is an elongated opening.

4. The handle assembly of claims 2-3, wherein the housing further includes a second axial opening (138) for receiving a first locking element (142).

5. The handle assembly of claim 4, wherein the housing further includes a third axial opening (140) for receiving a second locking element (144).

6. The handle assembly of claims 2-5, wherein the housing further includes a laterally facing counterbore (152).

7. The handle assembly of claims 1-6, wherein the quick connect comprises a locking mechanism (154) for releasably engaging with the shaft of the medical device instrument.

8. The handle assembly of claim 7, wherein the locking mechanism comprises:
a locking member (150) having first and second ends (158, 160) and an elongated through hole (162) for receiving the shaft of the medical device instrument; and
a biasing member (164) biasing the second end of the locking member.

9. The handle assembly of claims 7-8, wherein the locking member comprises a lateral recess (146).

10. The handle assembly of claims 7-8, wherein the locking member comprises a pair of opposing lateral recesses (146, 148).

11. The handle assembly of claims 1-10, wherein the first end of the hand grip includes the ring and a lateral most end of the ring extends further laterally from the shaft than a lateral most end of the second end of the hand grip.

12. The handle assembly of claims 1-11, wherein the ring has an overall diameter larger than an overall thickness of the hand grip.
